(19)

Europäisches
Patentamt
European
Patent Office
Office européen
des brevets

(11) **EP 2 645 280 A1**

(12) **EUROPEAN PATENT APPLICATION**

(43) Date of publication:
 **02.10.2013 Bulletin 2013/40**

(51) Int Cl.:
 ***G06F 19/00*** *(2011.01)*

(21) Application number: **12305392.8**

(22) Date of filing: **30.03.2012**

(84) Designated Contracting States:
 **AL AT BE BG CH CY CZ DE DK EE ES FI FR GB
 GR HR HU IE IS IT LI LT LU LV MC MK MT NL NO
 PL PT RO RS SE SI SK SM TR**
 Designated Extension States:
 **BA ME**

(71) Applicants:
 • **Ecole Polytechnique
  91120 Palaiseau (FR)**
 • **Université Pierre et Marie Curie - Paris 6
  75005 Paris (FR)**

(72) Inventors:
 • **Rosmej, Frank
  92000 NANTERRE (FR)**
 • **Aouad, Youcef
  93400 Saint-Ouen (FR)**

(74) Representative: **Pontet Allano & Associes
 Parc Orsay Université
 25, rue Jean-Rostand
 91893 Orsay Cedex (FR)**

(54) **A rapidly converging atomic physics method and medium to calculate complex non-equilibrium radiative properties of matter with spectroscopic precision.**

(57) A rapidly converging atomic physics method and medium to calculate complex non-equilibrium radiative properties of matter and spectral distributions with spectroscopic precision.

The invention concerns a method for efficiently performing a complex spectral distribution of a radiation source by determining populations of matter in atomic levels, the method comprising:

using a computer to perform the following:

- considering an exact atomic system (up to the fine structure level or even above) containing multiple excited levels "j" belonging to M manifolds $\{\alpha(1)\}...\{\alpha(M)\}$, numerous ground and single excited levels "k", and corresponding transition matrix elements,

- performing a reduced system RS from the exact atomic system wherein all j-levels belonging to the manifolds $\{\alpha(1)\}...\{\alpha(M)\}$ are lumped together to create one level for each manifold $\{\alpha(\beta)\}$, comprising of all possible methods for generalizing the manifold definition,

- performing an Independent Recovery System of Equations IRSE containing j recovery systems computed from the exact atomic system, each recovery system comprising, instead of all j levels, a level j and a level $m_j$ obtained by lumping together all levels from the manifolds, except the j-level itself,

- calculating transition matrix elements $W_{\alpha jk}$ and $W_{k\alpha j}$ (note, that $\alpha_j$ is the level number in RS of the manifold $\{\alpha\}$ to which j belongs) of RS to determine populations $n_k$ of all levels k,

- for each recovery system, calculating transition matrix elements $W_{mjk}$, $W_{km_j}$, $W_{mj}$ and $W_{jm_j}$ from $n_k$ to determine a non-statistical excited populations $n_j^{(\kappa)}$ of each level j ("$\kappa$" is the iteration index)

FIGURE 3

**Description**

**Technical field**

The present invention relates to the determining of radiative properties of matter like, e.g., plasmas, gases, solids. These radiative properties can be used to diagnose complex matter/plasma conditions and the spectral distribution characterizes the radiation loss in energy and frequency. In particular, the present invention concerns a method to calculate complex non-equilibrium radiative properties and spectral distributions of matter with high spectroscopic precision (corresponding to the fine structure level of atomic quantum mechanical wavefunctions or above).

**State of prior art**

Atomic populations kinetics of multiple excited states (autoionizing) is a key-point for the calculations of, e.g., atomic/ionic fractions, radiation losses and spectral distributions. The precise knowledge of the spectral distribution is, e.g., needed for the development of radiation sources and also for any diagnostics via emission spectroscopy (which is a non-perturbative method): applicable if the objet is not accessible because it is, e.g., too hot, too far, too shortly emitting etc...

The usually large number of atomic levels (thousands up to millions) for rather simple distributions of electrons over the atomic/ionic/molecular shells prevents precise calculations due to limited computational resources. The limitation concerns even the simplest stationary calculations implying that transient or even integrated simulations (means coupled, e.g., to simulations of the macroscopic evolution of matter "Hydro") are almost impossible.

The spectral distribution $I(\omega)$ (means the energy radiated per surface unit, in a certain direction, per solid angle, per second, per angular frequency interval: J/s·m²·sr·2πHz) is the most detailed key-parameter to describe the radiative properties of matter [H.R. Griem, Principles of Plasma Spectroscopy, Cambridge University Press, New York 1997]:

$$I(\omega) = \sum_{j=1}^{N} \sum_{i=1}^{N} \frac{\hbar \omega_{ji}}{4\pi} n_j A_{ji} \phi_{ji}(\omega) \qquad (1.1)$$

Here $n_j$ is atomic/ionic/matter population density of the upper level $j$, $A_{ji}$, $\hbar$ $\omega_{ji}$, and $\phi_{ji}(\omega)$ are the probabilities of radiative transition, transition energy and spectral line shape of the transition $j \rightarrow i$, respectively. $N$ is the total number of bound levels (ground states, single and multiple excited states) of all ionization stages.

The standard method for the calculation of the atomic level populations consists in solving an exact atomic system of kinetic differential equations in which all levels and all possible collisional-radiative processes are taken into account. In this model, the level-population densities $n_j$ satisfy the following equations (so called exact atomic system according to standard theory):

$$\frac{dn_j}{dt} = -n_j \sum_{m=1}^{N} W_{jm} + \sum_{n=1}^{N} n_n W_{nj} \qquad (1.2)$$

$W$ is the population matrix of all collisional and radiative transition rates connecting the discrete levels $j$ to all other levels in all ionization stages. If a particular transition $j \rightarrow i$ cannot occur due to physical principles $W_{ji} = 0$. The population matrix elements are given by:

$$W_{ij} = W_{ij}^{rad} + W_{ij}^{col} \qquad (1.3)$$

The matrix elements describing the radiative and autoionizing processes are given by

$$W_{ij}^{rad} = A_{ij} + \Gamma_{ij} + P_{ij}^{abs} + P_{ij}^{em} + P_{ij}^{rr} + P_{ij}^{iz} \qquad (1.4)$$

The collisional processes are described by

$$W_{ij}^{col} = n_e C_{ij} + n_e I_{ij} + n_e^2 T_{ij} + n_e R_{ij} + n_e D_{ij} \ldots \ldots \quad (1.5)$$

$A_{ij}$ : Spontaneous radiative decay rate
$\Gamma_{ij}$ : Autoionization rate

$P_{ij}^{abs}$ : Stimulated photoabsorption

$P_{ij}^{em}$ : Stimulated photoemission

$P_{ij}^{rr}$ : Stimulated radiative emission

$P_{ij}^{iz}$ : Photoionization

$C_{ij}$ : Electron collisional excitation/ deexcitation
$I_{ij}$ : Electron collisional ionization
$T_{ij}$ : 3- body recombination
$R_{ij}$ Radiative recombination
$D_{ij}$ : Dielectronic capture
The dots in eq. (1.5) indicate other - in principle possible - processes, like, e.g., charge exchange and heavy particle collisions.
Nowadays computer codes are freely available that allow the calculation of all necessary energy levels/ wavelengths, collisional and radiative transitions to establish the population matrix (eqs. 1.3-5) for almost all practical applications. This permits to solve numerically the system of equations (1.1, 1.2). A detailed discussion of the transition matrix elements and line profiles is not presented here, as this does not directly concern the present invention and methods are publically available for their efficient calculation, see for example: R.D. Cowan, The Theory of Atomic Structure and Spectra, University of California Press, Berkely 1981; H.R. Griem, Principles of Plasma Spectroscopy, Cambridge University Press, New York 1997; M.F. Gu, The flexible atomic code FAC, Canadian Journal of Physics 86 (5), 675 (2008).
Although equations (1.1) and (1.2) look rather transparent, the practical application is extremely difficult due to the necessity to include a large number of multiple excited (autoionizing) states to correctly describe:

1) all line transitions (from single and multiple excited states) belonging to a certain spectral range of interest,
2) the ionic charge state distribution that influences on all atomic populations ($n_j$ in eq. 1.1, 1.2) and, in consequence to the spectral distribution of any spectral interval,
3) all possible line transitions of all charge states to predict the radiation energy loss of a given system (means eq. (1.1) for frequencies ranging from zero to infinity).

Even for atoms with only a few numbers of atomic bound electrons the number of requested levels $N$ can be so large that the standard method (means to solve eqs. (1.1) and (1.2) for all levels that need to be taken into account for the precise description of the spectral distribution) turn out to be impractical even for a high level computational infrastructure.
The Document "A. Burgess, Astrophys. Journal 139, 776 (1964)" describes dielectronic recombination that is one of the most important process influencing on the charge state distribution of atoms and ions. Unfortunately the exact calculation of this process involves the implication of a so large number of levels that direct calculations had only be possible today for certain specific cases. Even for one of the simplest ions (namely He-like ions) the atomic configurations to incorporate dielectronic capture from He-like to Li-like ions requests about thousand states (namely the 1s2lnl'- and 1s3ln1"-configurations) to be included in the system (1.2). It is entirely impractical to include thousands and or even millions of states just for the purpose to calculate a certain type of transition matrix elements (namely $D_{ij}$, eq. (1.5)) to connect different charge states.
Let us consider a very simple example to illustrate the various atomic states involved, namely the dielectronic recombination from the H-like ground state 1s:

dielectronic capture :      $1s + e \rightarrow 2p^2 \, LSJ$
autoionization :             $2p^2 LSJ \rightarrow 1s + e$
dielectronic recombination :   $2p^2 LSJ \rightarrow 1s2p \, L'S'J' + \hbar\omega_{sat} \, 1s2p \, L'S'J' \rightarrow 1s^2 + \hbar\omega_{res}$

After dielectronic capture into the H-like ground state 1s, the multiple excited state $2p^2$ is formed (due to different possibilities of coupling orbital and spin quantum numbers of the two electrons numerous different states LSJ are formed from the simple $2p^2$ configuration). This state can either decay via a non-radiative decay (autoionization) or radiative decay (spontaneous radiative emission). For the case of autoionization of the state $2p^2$, the original state 1s is "returned" and no effective change in the charge state distribution occurs. If, however, the state $2p^2$ decays via a radiative decay (corresponding to a photon with energy $\hbar\,\omega_{sat}$) the state 1s2p is created which decays by another radiative decay (photon with energy $\hbar\,\omega_{res}$) that produces the state $1s^2$. Therefore, dielectronic capture following by successive radiative decays produces a different charge state. As the H-like charge state is transformed to a He-like charge state, this ensemble of atomic physics processes leads to an effective recombination that is called "dielectronic recombination". For the above example k=1s, j=$2p^2$ and i=1s2p (see eq. (1.6) below). All configurations 2l2l' contribute in a similar manner to the dielectronic recombination, but also 2lnl' and in general nln'l' which comprises already hundred of states. For more complex configuration in the L, M and N shell, similar consideration leads rapidly to millions of states that need to be considered to simulate the dielectronic recombination to get right only the charge state distribution.

Moreover, this example shows, that the dielectronic recombination influences also directly on the spectral distribution via spontaneous radiative transitions: the radiative decay of the so called "satellite photon" with energy $\hbar\,\omega_{sat}$ contributes to the spectral distribution and this contribution might be even larger than the usual resonance line emission of single excited states (photons with energy $\hbar\,\omega_{res}$ in the above example) .

Based on the theory of Burgess numerous approximations have been developed in order to calculate effective dielectronic recombination rates that avoid explicit incorporation of the multiple autoionizing states in eq. (2-5). In all these approximations it is, however, assumed that effective recombination from one charge stage to another is given by a branching ratio

$$B_{k,ji} = \frac{A_{ji}}{\sum_i A_{ji} + \sum_k \Gamma_{jk}} \qquad (1.6)$$

after dielectronic capture from state "k" to state "j". $A_{ji}$ is the spontaneous transition probability from level j to i and $\Gamma_{jk}$ is the autoionizing rate from level j to level k. Such a method is disclosed in R.D. Cowan, The Theory of Atomic Structure and Spectra, University of California Press, Berkely 1981.

Concerning our simple example, an effective dielectronic recombination rate couples the states 1s and $1s^2$ thereby avoiding the explicit incorporation of all the nln'l'-sates. This, however, neglects important collisional effects on atomic populations of the multiple excited states that is only well justified in very low-density plasmas (so called Corona-regime). However, even for rather low density plasmas, the complex level structure (that include meta-stable levels) in the L and, particularly, M and N atomic shells of high Z-elements play an important role. The use of effective dielectronic recombination rates even for low density plasmas is then highly questionable as numerous k-states are excited in a collisional radiative regime. In dense plasmas, a Burgess-type approximation is highly unsatisfactory due to the additional absence of further physical effects: ionization from the multiple excited states, collisional redistribution between the multiple excited states, important population of numerous excited states "k" (which can be comprised from ground, single and even multiple excited states).

Moreover, if charge exchange or photoionzation processes are important population processes for multiple excited states (for example in gas mixtures or for intense radiation sources) the multiple excited states and hollow ion configurations are *not* linked anymore by dielectronic recombination. Effective rate coefficients according the Burgess theory then entirely fail even in low- density plasmas to describe the situation.

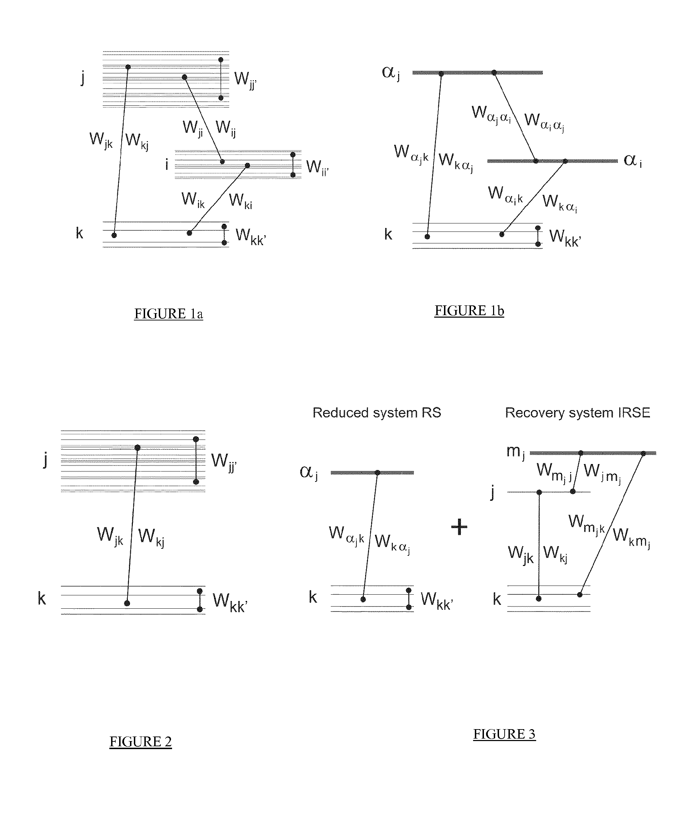

The Document "A. Bar- Shalom, J. Oreg, W.H. Goldstein, D. Shvarts, A. Zigler, Phys. Rev. A 40, 3183 (1989) ", discloses another approach to reduce the huge number of multiple excited states necessary for a precise calculation. This approach, so called Superconfiguration (SC) approach consists in replacing the numerous levels of one configuration by a reduced number of levels to obtain a manageable system (eqs. 1.2) of atomic levels. Figures 1a, b depict the basic principle of this approach. Fig. 1a shows an exact level system: depicted are two configurations of multiple excited states "j" and "i", k designates ground and single excited states (non- autoionizing) . Due to the large number of multiple excited states "j" and "i" the system of differential equations (1.2) is very large and prohibitive for numerical solution.

Fig. 1b shows an example of a reduced atomic level system. All levels "j" are lumped together into one level

$\alpha_j$ (note that $\alpha_j$ is the level number of the manifold $\{\alpha\}$ to which j belongs), all levels "i" are replaced by one level $\alpha_i$. The number of levels is therefore drastically reduced and the system of equations (1.2) is reduced to a manageable dimension for numerical calculations. This is the principle of the SC approach: $\alpha_j$ and $\alpha_i$ are the so-called SC's. In order to link the SC's to each other and to the levels "k", one needs also to lump together all detailed population matrix elements $W_{ij}$, $W_{ji}$, $W_{ki}$, $W_{ik}$, $W_{kj}$ and $W_{jk}$. This is represented in Fig. 1b by the matrix elements $W_{\alpha i \alpha j}$, $W_{\alpha j \alpha i}$, $W_{k \alpha i}$, $W_{\alpha i k}$, $W_{k \alpha j}$, and $W_{\alpha j k}$.

However, this reduction method has serious drawbacks:

a) As all levels "j" and all levels "i" are replaced by one level $\alpha_i$ and $\alpha_i$ respectively, the spectral distribution originating from the numerous transitions $j \rightarrow i$ is replaced by only one transition (with artificial energy/line center position). Therefore, all details of the original spectral distribution are destroyed and spectroscopic precision is entirely lost.

b) In order to calculate the effective matrix elements $W_{\alpha i \alpha j}$, $W_{\alpha j \alpha i}$, $W_{k \alpha i}$, $W_{\alpha i k}$, $W_{k \alpha j}$ and $W_{\alpha j k}$ one needs in fact to know the detailed populations of each level "j" and level "i" (to be discussed in detail below) requesting in turn the consideration of equations that are linked by the "internal" populations matrix elements $W_{jj}$, and $W_{ii}$, (see Fig. 1a). This, however, would request the exact solution of the level system according Fig. 1a and no advantage of the reduced system is obtained. Therefore numerous additional assumptions are introduced to the reduced system, for example a statistical population between all levels "j" and all levels "i", or a Boltzmann like distribution (designated also as LTE populations in the literature, for Local Temperature thermodynamic Equilibrium) with an artificial temperature (so-called SC-temperature). The precision of these assumptions are difficult to estimate, in fact, their strict justification would again require the solution of the exact atomic level system (Fig. 1a) which is supposed to be avoided. Numerous variations of the standard SC approach are developed. In short terms, these ideas are essentially based on the reduction of all levels "j" and "i" to numerous SC's to improve the above discussed deficiencies. But this means again that one drives the reduced system towards the exact atomic system (thereby introducing additional assumption how to create numerous SC's from all the levels "j" and "i").

## Description of the invention

An object of the present invention is to overcome drawbacks of prior art by providing an accelerated method to determine complex non-equilibrium (transient, non-LTE, non-Maxwellien) radiative properties of matter with spectroscopic precision.

Another object of the present invention is to provide a highly convergent method.

In at least preferred embodiments, the present invention provides a method for efficiently determining a complex spectral high resolution distribution of a radiation source by determining populations of matter in atomic levels, the method comprising:

using a computer to perform the following:

- considering an exact atomic system, up to the fine structure level or even above, containing multiple excited levels "j" belonging to $M$ manifolds $\{_\alpha (1)\}$ ...$\{_\alpha (M)\}$, numerous ground and single excited levels "k", and corresponding transition matrix elements,
- performing a reduced system RS from the exact atomic system wherein all j- levels belonging to the manifolds $\{_\alpha (1)\}$ ...$\{_\alpha (M)\}$ are lumped together to create one level for each manifold $\{_\alpha (\beta)\}$, $\beta$=1 to M
- performing an Independent Recovery System of Equations IRSE containing j recovery systems computed from the exact atomic system, each recovery system comprising, instead of all j levels, a level j and a level $m_j$ obtained by lumping together all levels from the manifolds, except the j-level itself,
- calculating transition matrix elements $W_{\alpha j k}$ and $W_{k \alpha j}$ of RS to determine populations $n_k$ of all levels k,
- for each recovery system, calculating transition matrix elements $W_{mjk}$, $W_{kmj}$, $W_{mjj}$ and $W_{jmj}$ from $n_k$ to determine a non-statistical excited populations $n_j^{(\kappa)}$ of each level j, "$\kappa$" being an iteration index $\geq 0$.

According to the invention, during the step of calculating transition matrix elements, all variations of initial levels population assumptions (means for $\kappa$=0) including Boltzmann, non-Boltzmann and combinations of Boltzmann and non-Boltzmann initial assumptions may be considered. $\kappa$=0 means the "0"-order solution in an n-order iteration scheme (see below) of each level j.

Concerning the manifold, the invention relates to all possible methods for generalizing the manifold definition including ground and single excited levels k and even mixed manifolds (containing k and j levels).

Note that $\alpha_j$ is the level number in RS of the manifold $\{\alpha\}$ to which j belongs.

The present invention develops a new atomic population kinetics and numerical method that, unlike the standard theory, has no need to establish the large exact atomic system that is prohibitive for numerical solutions. Compared to the exact atomic system that contains a huge number of levels coupled via differential equation like eq. (1.1), the number of levels in the RS is highly reduced to a numerically manageable number and one equation of the IRSE is a two level system that allows even an analytical solution. It is therefore advantageous to perform several small size matrices than a few huge size matrices.

Convergence to the exact solution is obtained with two highly reduced systems and an indirect coupling via a transition matrix. Due to the extremely high convergence properties (even the 0-order is already rather precise) the most complex phenomena related to the radiative properties of matter become accessible via numerical simulations with high spectroscopic precision. This will allow design and optimization studies of almost all radiation sources and diagnostic developments.

The method is applicable to all kind of atomic configurations, in particular to the most complex ones like autoionizing states, multiple excited states, hollow ion/atom states and any combination of hole states. The generality of the method combined with the extreme convergence properties to high spectroscopic precision permits also integrated simulations, e.g., hydro simulations, particle kinetic simulations, that have not been possible up to date.

According to the invention, the method further comprises iterative steps from index $\kappa=0..i..i_{max}$, with "$i_{max}$" being the number of iterations, of:

- inserting simultaneously into RS and IRSE the non-statistical excited populations $n_j^{(i-1)}$, comprising all variations of initial levels population assumptions (means for $\kappa=0$) including Boltzmann and non-Boltzmann and combinations of Boltzmann and non-Boltzmann,

- refining calculation of transition matrix elements $W_{\alpha jk}$ and $W_{k\alpha j}$ of RS from the non-statistical excited populations $n_j^{(i-1)}$,

- refining calculation of transition matrix elements $W_{mjk}$, $W_{kmj}$, $W_{mjj}$ and $W_{jmj}$ of IRSE from the non-statistical excited populations $n_j^{(i-1)}$.

The invention comprises a new splitting technique of atomic levels combined with a new split-iteration numerical procedure. This allows acceleration of numerical calculations by many orders of magnitude and permits simulating even the most complex non-equilibrium radiative properties of matter.

The iteration steps concern a split-iteration numerical method wherein the iterations RS and IRSE are performed simultaneously in parallel. This permits a highly convergent procedure.

The present invention allows reducing considerably the requested computational resources in all fields where radiation properties are needed. It might also be interesting for lamp/radiation source production industry to predict radiation loss and detailed spectral distributions (which is, e.g., the quantity to estimate the "quality" of the radiation source, like "cold light", "warm light"). The method of the invention is therefore also highly interesting for non-stationary radiation source development (e.g., radiation sources for microprocessor production by lithography, pulsed power radiation sources driven, e.g., by lasers, pinch plasmas).

According to the invention, after each iteration, the method further comprises determining if the non-statistical excited populations $n_j^{(i-1)}$ have converged, for example by considering numerical values of $n_j^{(i-1)}$, $n_j^{(i)}$ and $n_j^{(i+1)}$.

According to the invention, the iteration is accelerated by calculating refined populations $n_j^{(i)}$ not only from $n_j^{(i-1)}$ but from several preceding populations, like, e.g. from $n_j^{(i-1)}$, $n_j^{(i-2)}$, ....

Advantageously, during the calculations of lumped transition matrix elements $W_{\alpha jk}$, $W_{k\alpha j}$, $W_{kmj}$, $W_{mjj}$ and $W_{jmj}$, in every iteration step requested non-lumped transition matrix elements $W_{xy}$ and $W_{yx}$ (where "x" and "y" designate any level x, y=1..N from the exact atomic system described by eq. (1.2) ) are known from pure non-lumped atomic physics calculations beforehand. Therefore, no "atomic physics calculation effort" is requested in the preceding iteration steps and iteration itself is therefore extremely fast. For each iteration, the method further comprises determining weather the matrix elements and the level $m_j$ have converged.

In this respect, the invention also enables the provision of a new non-transitory computer-readable storage medium encoded with computer-executable instructions which, when executed, perform a method for determining complex spatial distribution of a radiation source by determining populations of matter in atomic levels, the method comprising the steps of:

- considering an exact atomic system containing multiple excited levels "j" belonging to **M** manifolds {α (1) } ... {α (**M**) }, numerous ground and single excited levels "k", and corresponding transition matrix elements,
- performing a reduced system RS from the exact atomic system wherein all j- levels belonging to the manifolds {α (1) } ... {α (**M**) } are lumped together to create one level for each manifold {α (β) }, β=1 to M
- performing an Independent Recovery System of Equations IRSE containing j recovery systems computed from the exact atomic system, each recovery system comprising, instead of all j levels, a level j and a level $m_j$ obtained by lumping together all levels from the manifolds, except the j-level itself,
- calculating transition matrix elements $W_{\alpha jk}$ and $W_{k\alpha j}$ of RS to determine populations $n_k$ of all levels k,
- for each recovery system, calculating transition matrix elements $W_{mjk}$, $W_{kmj}$, $Wm_{jj}$ and $W_{jmj}$ from $n_k$ to determine a non-statistical excited populations $n_j^{(\kappa)}$ of each level j, "κ" being an iteration index ≥0

The new splitting technique allows convergence to the exact solution for any plasma parameters (in particular non-LTE) with a highly reduced system that has the outstanding advantage to avoid establishing the entire exact atomic population kinetic system even for convergence studies. Moreover, it has been shown that the 0-order solution (κ=0, equivalent to an analytic solution) is already of very high quality (spectroscopic precision). Therefore, also transient non-equilibrium radiative properties (e.g., radiation loss calculations, ionic populations for different charge states, spectral distributions of high spectroscopic precision that allows detailed diagnostics) can be additionally accelerated by many orders of magnitude.

**Description of the figures and embodiments**

For the purpose of illustrating the invention, there is shown in the drawings a form that is presently preferred; it being understood, however, that this invention is not limited to the precise arrangements and instrumentalities.

Figures 1a and 1b respectively illustrate an exact atomic level system and a reduced atomic level system according to prior art,
Figure 2 is a view illustrating an exact atomic level system for one configuration of levels "j",
Figure 3 is a view illustrating a reduced atomic level system and one of the j equations of a new Independent Recovery System of equations according to present invention,
Figures 4a and 4b are graphs illustrating the spectral distribution ("spectra") for titanium Z=22 for 0-iteration (κ=0) and 1-iteration (κ=1),
Figures 5 is a bloc diagram of an exemplary computer system for implementing a registration method according to the present invention.

While the invention is susceptible to various modifications and alternative forms, specific embodiments thereof are shown by way of example in the drawings and will herein be described in detail. It should be understood, however, that the drawings and detailed description thereto are not intended to limit the invention to the particular form disclosed, but on the contrary, the intention is to cover all modifications, equivalents, and alternatives falling within the spirit and scope of the present invention as defined by the appended claims.
On Figure 2 only one configuration $\alpha_j$ that contains the levels "j" is presented (the extension to many configurations, like, e.g., presented in Fig. 1a is straightforward but will considerably prevent a transparent presentation).
The new atomic physics splitting technique works in the following way: first, the exact atomic system (Fig. 2) of equations (1.2) is transformed to a **R**educed **S**ystem "RS" (Fig. 3 left) lumping levels "j" to one level "$\alpha_j$", second, every level "j" (which does not anymore explicitly appear in the RS) is recovered in an **I**ndependent **R**ecovery **S**ystem of **E**quations "IRSE" (Fig. 3 right). The number of recovery systems (Fig. 3 right) is exactly identical to the number of all levels "j" in the exact atomic system (Fig. 2). The level "$m_j$" in the recovery system is a level that is obtained when lumping together all levels from which "$\alpha_j$" is composed, except the j-level itself (for example, if the configuration "$\alpha_j$" is composed from 10 levels, means j=1...10 and the recovery system for the level j=3 is considered, the level $m_j$ is composed of the levels [1, 2, 4, 5, 6, 7, 8, 9, 10]). The levels "k" in the exact atomic system (Fig. 2), in the RS and in the IRSE are the same (as indicated in Fig. 3).
It is already visible at this point that the IRSE is not a usual system of equations according to the standard theory nor to the SC-approach (that contains N-equations for N levels, like, e.g., equations (1.2)) but rather a hierarchic system of equations where each single IRSE-equation contains only the level "j" that is coupled indirectly to all other levels via the transition matrix elements W (see Fig. 3, right). The hierarchy manifests itself by the fact that the IRSE is under the control of RS (due to the levels "k") whereas IRSE returns detailed information of level "j" to RS via the matrix elements $W_{\alpha jk}$ and $W_{k\alpha j}$. Due to the hierarchic communication of the two systems, RS + IRSE contain therefore information for all single levels "j" and level k like the exact atomic system (Fig. 2). However, compared to the exact atomic system that contains a huge number of levels coupled via a set of differential equations (eq. 1.2), the number of levels in the RS is highly reduced to a numerically manageable number and one equation of the IRSE is a two level system that allows even an analytical solution.

What remains to establish is the hierarchic link of RS and IRSE via the transition matrix elements $W_{\alpha jk}$, $W_{k\alpha j}$, $W_{jk}$, $W_{kj}$, $W_{mjk}$, $W_{kmj}$, $W_{mjj}$ and $W_{jmj}$ (see Fig. 3) that will allow obtaining exact populations for each level "j" and "k" like the highly inconvenient exact atomic system according Fig. 2. This is realized with the highly convergent split-iteration numerical procedure.

The new highly convergent split-iteration numerical procedure can be described as follows.

The split iteration numerical procedure is a double fold interaction: an external iteration that couples RS and IRSE and an internal iteration (inside IRSE) that couples the level $m_j$ to the level j for each IRSE(j). The highly convergent aspect of the present invention is that this numerical procedure avoids performing one iteration after another (which increases the numerical burden considerably) but rather allows a simultaneous iteration of "internal + external iteration" without loss of convergence in each iteration step.

The new method works as follows. Start with the system RS assuming, for example a Boltzmann distribution of all levels "j" to calculate the transition matrix elements $W_{\alpha jk}$ and $W_{k\alpha j}$ (note that also other assumptions can be made at this step). The solution of RS provides the populations of all levels "k". In order to solve one equation of IRSE, namely IRSE(j), the populations of the levels "k" from RS are inserted in IRSE(j) (for this reason no transition matrix of the type $W_{kk}$, is indicated in Fig. 3 for IRSE) combined also with a Boltzmann distribution of all levels from which "$m_j$" is composed that provides in turn the requested transition matrix elements $W_{mjk}$, $W_{kmj}$, $W_{mjj}$ and $W_{jmj}$.

The transition matrix elements $W_{jk}$, $W_{kj}$ are known from pure atomic physics calculations beforehand (note that no level lumping and populations is involved for these levels). The solution of the "j's" IRSE equation (denoted like IRSE (j)) provides a non-statistical population of level "j" because the coupling of "j" to $m_j$ and k is exact except the assumption for the matrix elements $W_{mjk}$, $W_{kmj}$, $W_{mjj}$ and $W_{jmj}$. This is called the 0-iteration non-LTE population of level "j": $n_j^{(\kappa=0)}$. The procedure for IRSE is repeated for each level "j" from which the exact atomic system is composed providing then the whole set of populations $n_j^{(\kappa=0)}$.

The next step is the implementation of the new double fold iteration (called split-iteration numerical procedure in the following): the new non-LTE populations $n_j^{(\kappa=0)}$ are inserted simultaneously into the system RS (to refine the transition matrix $W_{\alpha jk}$ and $W_{k\alpha j}$ that become non-LTE) and into all equations of IRSE (to refine $W_{mjk}$, $W_{kmj}$, $W_{mjj}$ and $W_{jmj}$, that become also non-LTE) rather than performing a separate iteration for each IRSE(j) to enter a converged result to RS.

This new split-iteration numerical procedure is demonstrated to be so highly convergent that even the 0-order solution (which is demonstrated below to be transformable to an analytical solution) allows treating the most complex problems in radiation physics with spectroscopic precision. This allows exploring cases that have never been possible before.

Below, the detailed set of equations is developed that can be used to implement the present invention for numerical calculations.

Also a prototype is developed that allows demonstration of the present invention via one of the most difficult atomic physics cases for purposes of the present invention.

**Detailed radiation physics description to implement the present invention**

We start from the basic principles in atomic population kinetics (system of equations (1.2)) and compose in the system "RS" the total number of levels $N$ into two parts $N_b$ and $N_a$ : the total numbers of ground + single excited levels $N_b$ and multiple excited/autoionizing levels $N_a$ (note that also other type of manifolds can be defined including even mixed ones composed from levels belonging to the groups $N_b$ and $N_a$).

$$N = N_b + N_a \qquad\qquad (3.1)$$

The total number of levels $N_a$ is distributed into M manifolds $\{_\alpha (1) \}$ .... $\{\alpha (M) \}$ with the number of specific

levels $N_{\alpha(\beta)}$ ($\beta$=1...M) in each manifold:

$$N_a = \sum_{\beta=1}^{M} N_{\alpha(\beta)} \qquad (3.2)$$

As an example, one can consider the case of He- alpha ($1s^2\,{}^1S_0$- $1s2p\,{}^1P_1$) satellites from lithium- like ions. The manifolds are of the form: {$\alpha$ (1) } = {$1s2l2l'$} with $N_{\alpha(1)}$ = 16, {$_\alpha$ (2) } = {$1s2l3l'$} with $N_{\alpha(2)}$=66 etc. With the help of these configurations it is possible to reduce the number of equations of the exact atomic system (eq. (1.2) ) by using manifold populations $n_\alpha$ in the kinetic equations of RS, instead of explicit implementation of all atomic levels "j" belonging to each manifold {$\alpha$} . Thus the total number of equations of RS $N^r$, given by $N^r=N_b+M$, is substantially decreased in comparison to the exact atomic system according standard theory containing $N = N_b +N_a$ coupled equations.

In order to demonstrate the technique of the present invention, the case of one manifold is considered (Figs. 2 and 3). In this case the reduced number of equation $N^r$ becomes equal to $N_b + 1$ in RS (Fig. 2). The population averaged excitation $\overline{W}_{k\alpha}$ and depopulation $\overline{W}_{ak}$ atomic rates for the manifold {$\alpha$} are introduced. This average is given by:

$$\overline{W}_{k\alpha} = \sum_{j\in\{\alpha\}} W_{kj} \qquad (3.3)$$

$$\overline{W}_{\alpha k} = \frac{\displaystyle\sum_{j\in\{\alpha\}} n_j W_{jk}}{\displaystyle\sum_{j\in\{\alpha\}} n_j} \qquad (3.4)$$

In Eqs. (3.3, 3.4) the sum is over all specific sublevels $j$ entering the manifold {$\alpha$}, $n_j$ is the population of the level $j$, whilst $W_{jk}$ and $W_{kj}$ are the transition rates (that are known beforehand, see paragraph II.) of the specific transitions between $k$ and $j$. The populations of the manifold {$\alpha$} satisfy the normalization condition $n_\alpha = \sum_{j\in\{\alpha\}} n_j$ and the reduced set of kinetic equations of RS is given by:

$$\begin{cases} \dfrac{dn_k}{dt} = -n_k \left( \displaystyle\sum_{k'=1}^{N_b} W_{kk'} + \overline{W}_{k\alpha} \right) + \displaystyle\sum_{k'=1}^{N_b} n_{k'} W_{k'k} + n_\alpha \overline{W}_{\alpha k} \ , \quad k = \left[1, N_b\right] \\[4mm] \dfrac{dn_\alpha}{dt} = -n_\alpha \displaystyle\sum_{k=1}^{N_b} \overline{W}_{\alpha k} + \displaystyle\sum_{k=1}^{N_b} n_k \overline{W}_{k\alpha} \end{cases} \qquad (3.5)$$

As introduced before, in RS according eq. (3.5), $k$ and $k'$ denote ground and single excited bound states. Therefore, for the specific example considered here, there exist $N_b$ equations for the bound states $k$ and one equation governing the manifold {$\alpha$}. Considering RS only the spectral distribution from the manifold {$\alpha$} (due to different bound levels $k$) is given by:

$$I_\alpha = n_\alpha \sum_{k=1}^{N_b} \frac{\hbar\omega_{\alpha k}}{4\pi} \overline{A}_{\alpha k} \phi_{\alpha k}(\omega) \qquad (3.6)$$

$n_\alpha$ is the population of the manifold alpha obtained from the system of equations Eq. (3.5) and $\hbar\omega_{\alpha k}$ is the averaged energy between the manifold alpha and the specific level $k$ . $\phi_{\alpha k}$ is the profile of the transition $\alpha \to k$ and $A_{\alpha k}$ is the averaged radiative transition rate of the transition in question. Eq. (3.6) demonstrates that several transitions from specific levels belonging to the manifold alpha are replaced by only one artificial transition from the configuration alpha. As discussed above, the restriction to the RS does not provide the requested exact transition matrix elements that need the knowledge about the exact populations $n_j$ (see eq. 3.4). Therefore the normalization condition $n_\alpha = \sum_{j \in \{\alpha\}} n_j$ cannot be fulfilled and even the total radiation output that is determined via the population $n_\alpha$ is NOT correct. Moreover, all details of the spectral distribution are lost due to the introduction of artificially created transitions that are highly reduced in number and aligned with incorrect wavelengths. This is extremely harmful for the analysis of different physical phenomena occurring in the plasma that are sensitive to the spectral distribution (or in other words diagnostic application of such a reduced spectral distribution is highly questionable as spectroscopic precision is not maintained).

The power of the present invention is that it provides a solution to the problem of the calculation of spectral lines/spectral distribution without any loss of generality and spectroscopic precision. In the present method, to solve the system of eq. (3.5), a non-trivial solution $\tilde{n}_j$ is introduced via IRSE for each specific level $j$ belonging to alpha in order to calculate the averaged rates from eq. (3.4).

The difference between $n_j$ (exact atomic system ES) and $\tilde{n}_j$ (IRSE) is that in the ES that describes the population $n_j$, $j$ interacts with all the $N_b$ bound levels and directly with all the $N_a$-1 autoionization levels. But inside IRSE, that describes the populations $\tilde{n}_j$, $j$ interacts also with all the $N_b$ bound levels, but the interaction with the $N_\alpha$-1 autoionization levels is indirect and described by means of one effective level denoted as $m_j$ as shown in Fig. 3 (right). By construction, $m_j$ is an effective level, containing all levels of the manifold alpha except the level $j$, for which it is introduced. An effective level $m_j$ is defined for each autoionization level $j$ belonging to $\{\alpha\}$. The populations $\tilde{n}_j$ and $\tilde{n}_{m_j}$ of level $j$ and $m_j$ are described by the reduced kinetic system of two equations (means for each level j there are two equations for each IRSE(j)), that is derived introducing the relaxation matrix elements averaged, over all specific autoionization levels, belonging to $m_j$. $\overline{W}_{smj}$ and $\overline{W}_{mjs}$ designate the transition matrix elements while the level $s$ could be bound "k" or autoionization level $j$ (see IRSE in Fig. 3). These averaged rates describe the collisionnal-radiative processes like $\overline{A}_{mjs}$ and $\overline{A}_{smj}$ for radiative transitions and $\overline{C}_{mjs}$ and $\overline{C}_{smj}$ for collision etc. Similar to equations (3.3-4):

$$\overline{W}_{sm_j} = \sum_{\substack{j' \in m_j \\ j' \neq j}} W_{sj'} \qquad\qquad (3.7)$$

$$\overline{W}_{m_j s} = \frac{\displaystyle\sum_{\substack{j' \in m_j \\ j' \neq j}} \tilde{n}_{j'} W_{j's}}{\displaystyle\sum_{\substack{j' \in m_j \\ j' \neq j}} \tilde{n}_{j'}} \qquad\qquad (3.8)$$

In the two level system (IRSE(j)) that describes the evolution of $\tilde{n}_j$ interacting with $\tilde{n}_{mj}$ and $n_k$, it is advantageous to write down explicitly certain relaxation channels: radiative decay rates (A), autoionization rates (F), collisional excitation and deexcitation (C), other relaxation channels are designated as (X) (like photo and collisional ionization rates, three body recombination rates, charge exchange, etc.). The two level systems of reduced kinetic equations IRSE(j) for the autoionization states takes then the form:

$$\begin{cases} \dfrac{d\tilde{n}_j}{dt} = -\tilde{n}_j\left(\sum_i A_{ji} + \sum_t \Gamma_{jt} + n_e \overline{C}_{jm_j} + \overline{A}_{jm_j} + \sum_{X,k} X_{jk}\right) \\ \qquad + \tilde{n}_{m_j}\left(n_e \overline{C}_{m_j j} + \overline{A}_{m_j j}\right) + \sum_\gamma \sum_k n_k \gamma_{kj} \\ \dfrac{d\tilde{n}_{m_j}}{dt} = -\tilde{n}_{m_j}\left(\sum_i \overline{A}_{m_j i} + \sum_t \overline{\Gamma}_{m_j t} + n_e \overline{C}_{m_j j} + \overline{A}_{m_j j} + \sum_{X,k} \overline{X}_{m_j k}\right) \\ \qquad + \tilde{n}_j\left(n_e \overline{C}_{jm_j} + \overline{A}_{jm_j}\right) + \sum_\gamma \sum_k n_k \overline{\gamma}_{km_j} \end{cases} \qquad (3.9)$$

In the right hand side of the system of eqs. (3.9) $n_k$ are the populations of the bound atomic levels $k$ from RS, that drive the excitation channels with rates $\gamma$ for the levels $j$ and $m_j$ (e.g., like collisions (C), dielectronic capture (DC), three body recombination (TR)). The stationary solution of the system of eq. (3.9) is analytical and given by:

$$\tilde{n}_j = \frac{\displaystyle\sum_\gamma \sum_k \left\{\left(\sum_i \overline{A}_{m_j i} + \sum_t \overline{\Gamma}_{m_j t} + n_e \overline{C}_{m_j j} + \overline{A}_{m_j j} + \sum_{X,k} \overline{X}_{m_j k}\right)\gamma_{kj} + \left(n_e \overline{C}_{m_j j} + \overline{A}_{m_j j}\right)\overline{\gamma}_{km_j}\right\} n_k}{\Delta_j}$$

$$(3.10)$$

$$\Delta_j = \left(\sum_i A_{ji} + \sum_t \Gamma_{jt} + n_e \overline{C}_{jm_j} + \overline{A}_{jm_j} + \sum_{X,k} X_{jk}\right)\left(\sum_i \overline{A}_{m_j i} + \sum_t \overline{\Gamma}_{m_j t} + n_e \overline{C}_{m_j j} + \overline{A}_{m_j j} + \sum_{X,k} \overline{X}_{m_j k}\right)$$
$$-\left(n_e \overline{C}_{m_j j} + \overline{A}_{m_j j}\right)\left(n_e \overline{C}_{jm_j} + \overline{A}_{jm_j}\right)$$

$$(3.11)$$

The coupling of RS and IRSE is now done with a specific iteration procedure. To compute the averaged rates from eqs. (3.3, 3.4, 3.7, 3. 8), the first iteration step invokes an assumption for the autoionization levels j that belong to $\{\alpha\}$ (for example, start with a LTE-population inside alpha, but also other assumption are possible. It is important to note that these assumptions concern just the start of the interation procedure and are rapidly abandoned as iteration proceeds).

The results from eqs. (3.3, 3.4), $\overline{W}_{k\alpha}^{(0)}$ and $\overline{W}_{\alpha k}^{(0)}$ (note that $\overline{W}_{k\alpha}^{(0)}$ and $\overline{W}_{\alpha k}^{(0)}$ are LTE transition matrix elements due to the start with the Boltzmann assumption) are used to solve the reduced system of kinetic equations according eq. (3.5). This in turn provides the corresponding bound populations for the levels k: $n_k^{(0)}$ (note that $n_k^{(0)}$ are already non-LTE populations). The results from eq. (3.7-8), $\overline{W}_{sm_j}^{(0)}$ and $\overline{W}_{m_j s}^{(0)}$ (note that $\overline{W}_{sm_j}^{(0)}$ and $\overline{W}_{m_j s}^{(0)}$ are LTE transition matrix elements due to the start with the Boltzmann assumption) and the populations $n_k^{(0)}$ are then used in the equations eq. (3.10-11) to calculate the populations of the levels j of each IRSE(j): $\tilde{n}_j$. Due to the advantageous particular construction of RS+IRSE non-LTE results for the levels j are already obtained in the first step. In other words $\tilde{n}_j^{(\kappa=0)}$ is non-LTE. This first step of calculation is called 0-iteration ($\kappa$=0).

The numerical calculations (presented via a developed prototype below) show that the 0-iteration provides already a high spectroscopic precision that allows further application for transient/time dependent simulations of the present invention.

For the first or higher iterations, after 0-iteration, the populations $\tilde{n}_j^{(\kappa=0)}$ are inserted simultaneously into eq. (3.4) to replace $n_j$ and in eq. (3.8) to replace $\tilde{n}_j$, to calculate non-LTE transition matrix elements: in other words

$\overline{W}_{k\alpha}^{(1)}$, $\overline{W}_{\alpha k}^{(1)}$, $\overline{W}_{sm_j}^{(1)}$ and $\overline{W}_{m_js}^{(1)}$ are non-LTE transition matrix elements. The solution of RS provides improved populations for the levels "k", namely $n_k^{(1)}$. Employing the populations $n_k^{(1)}$ and transition matrix elements $\overline{W}_{sm_j}^{(1)}$ and $\overline{W}_{m_js}^{(1)}$ for each IRSE(j) according eqs. (3.10, 3.11) provides improved populations $n_j^{(1)}$. This is called 1-iteration ($\kappa=1$). Numerical calculations show, that the convergence properties of the new split-iteration numerical procedure are so high, that $n_j^{(1)}$ is already very close to the exact solution $n_j$, or in other words

$$\lim_{\kappa \to \infty} \left\{ \tilde{n}_j^{(\kappa)} \right\} = n_j \approx \tilde{n}_j^{(1)}. \qquad (3.12)$$

As the whole set of IRSE(j) provides all detailed populations "j" from the configuration "alpha" a detailed spectral distribution can be calculated by performing a sum over all specific levels belonging to the manifold alpha while the spectroscopic precision is preserved (which is lost when considering the system RS only, Figure 1b):

$$I_\alpha^{(\kappa)} = \sum_{j=1}^{N_a} \sum_{k=1}^{N_b} \hbar \omega_{jk} \, \tilde{n}_j^{(\kappa)} A_{jk} \, \phi_{jk}(\omega) \qquad (3.13)$$

Advantageously, the convergence studies are accessible via the numerical values of $\tilde{n}_j^{(\kappa)}$ and not, like in standard approaches, via the comparison with more and more refined atomic systems. Therefore, at no step of the numerical simulation the exact solution is requested. This has outstanding advantages for extremely complex cases where the exact atomic system cannot even be established (even not for a few benchmark cases to test the approximations made) .

## Development of a prototype

A prototype and successful demonstration of the present invention has been developed for a rather difficult case: numerical calculation of the spectral distribution originating from the multiple excited autoionizing levels *1s2l2l'* (lithium- like satellites of the He- alpha resonance line $1s^2$ $^1S_0$- $1s2p$ $^1P_1$ and intercombination line $1s^2$ $^1S_0$- $1s2p$ $^3P_1$) . Note, that the difficulty of the 1s2l2l'- configuration arises not so much from the number of levels but from the fact that dielectronic capture and innershell excitation channels are well separated in a high resolution spectral distribution. Therefore any inaccuracy in the present invention is directly visible (and is not masked by line overlap and overlap of different excitation channels) . That provides a stringent test of the method. Moreover, the 1s2l2l'- configuration emits also complex intercombination and two electron transitions that are usually very difficult to handle in atomic population kinetics: also these transitions are well separated from the others and imprecisions would likewise be immediately detected in the spectral distribution. Therefore, the chosen test case is well suited to test the present invention.

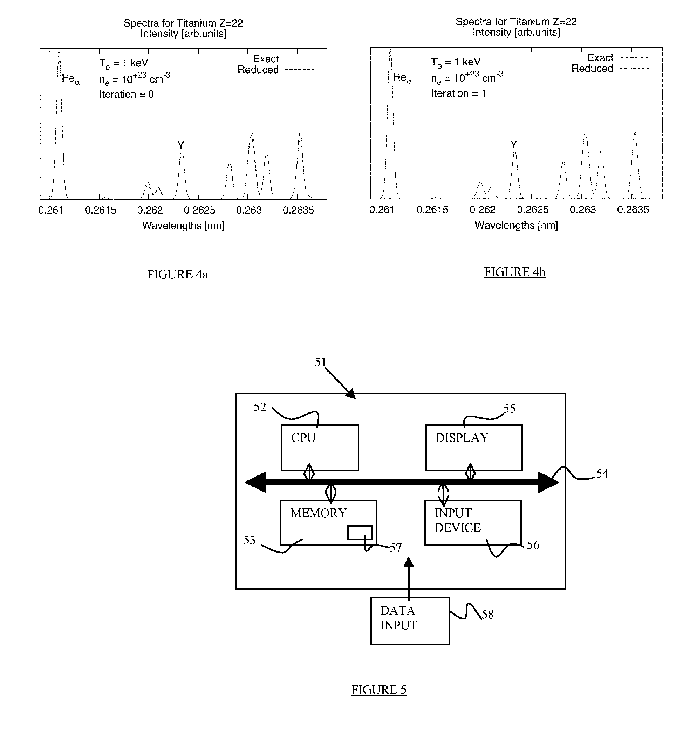

Figures 4a and 4b concern the titanium as a test element (nuclear charge Z=22). In the spectra, a comparison is made between the exact (solid curves) numerical calculations from eq. (1.2) and the analytical reduced kinetic model (dashed curves). The spectra are obtained using Gaussian profiles in order not to mask the comparison with line broadening effects for different densities.

Figures 4a and 4b show the comparison of exact and (RS+IRSE) for an electron temperature $T_e$=1keV, electron density $n_e$=10$^{+23}$ cm$^{-3}$ and different number of iterations. The chosen density corresponds to an intermediate regime between Corona and Boltzmann to critically test also the collisional-radiative regime (which is the most difficult to treat). Fig. 4a shows the results for the "0-iteration," case. One can see that the 0-iteraction provides already a very good approximation to the exact calculations. Fig. 4b demonstrates the high convergence properties of the new split iteration numerical procedure. The result for the "1-iteration," case is almost identical to the exact solution providing proof of eq. (3.12).

Table 1 illustrates detailed convergence studies for a broad range of plasma parameters presenting the ratio R that is defined by

$$R^{(\kappa)} = \frac{\sum\limits_{i=1}^{N}\sum\limits_{j=1}^{N} I_{ji}^{(\kappa)}\left(RS + IRSE\right)}{\sum\limits_{i=1}^{N}\sum\limits_{j=1}^{N} I_{ji}\left(exact\right)} \qquad (3.14)$$

where $I_{ji}^{(\kappa)}\left(RS + IRSE\right)$ is calculated according eq. (3.13) and $I_{ji}$ *(exact)* according eq. (1.1). As one can see from table 1, the "0-iteration," case provides already an excellent description of the exact results and confirms its importance in the framework of a numerical procedure with spectroscopic precision. Table 1 shows further, that the "1-iteraction" case provides already near exact results confirming the hypothesis of eq. (3.12) for all ranges of densities (note that the density interval chosen extends from the low density - Corona- until high density - Boltzmann- regime: this means that higher or lower densities than those depicted in table 1 do not change R for the lowest and highest densities depicted in table 1, table 1 covers therefore all possible density cases).

For high densities, all levels are populated according to Boltzmann and the "0-interaction" case is exact because a Boltzmann hypothesis has been considered for the transition matrix elements $\overline{W}_{k\alpha}^{(0)}$, $\overline{W}_{\alpha k}^{(0)}$, $\overline{W}_{sm_j}^{(0)}$ and $\overline{W}_{m_j s}^{(0)}$. In the opposite case, means for low densities, the atomic populations are very far from Boltzmann. Nevertheless table 1 shows the impressive result that even for this extreme case the "0-interaction" case is rather close to the exact result. This is due to the new highly efficient atomic physics splitting technique (Fig. 3) of the present invention which provides immediately a highly corrected non-LTE case.

*__Table 1:__ Test of convergence of iterations for titanium and for a fixed electronic temperature with four values of electronic densities to cover all regimes from corona, collisional-radiative to Boltzmann.*

Table 1: Titanium (Z=22) and $T_e$ =1 keV

| Iteration: N° | 0 | 1 | 2 | 3 | 10 |
|---|---|---|---|---|---|
| Ne = 1.E+10 cm-3 | 0,993 | 0,999 | 0,999 | 0,999 | 0,999 |
| Ne = 1.E+20 cm-3 | 0,978 | 0,999 | 0,999 | 0,999 | 0,999 |
| Ne = 1.E+23 cm-3 | 0,957 | 0,976 | 0,984 | 0,988 | 0,998 |
| Ne = 1.E+25 cm-3 | 1.000 | 1.000 | 1.000 | 1.000 | 1.000 |

The "0-iteration," case provides already a very high spectroscopic precision. As the 0-iteraction is equivalent to an analytical solution the present invention can directly accelerate transient calculations to spectroscopic precision. Assume that the time dependent evolution of a system is driven by the time dependent plasma parameters $n_e$ $(t_1)$ and $T_e(t_1)$. The time $t_t$ indicates each time step in the transient simulation. Application of the 0-iteration leads to the following advantageous description. At time step $t_J$ the 0-iteration solution of the present invention provides the populations $\tilde{n}_j^{(0)}\left(t_i\right)$ that has been demonstrated (Figures 4) to be of spectroscopic precision. These populations are used directly for the next time step $t_{i+1}$ to calculate the transition matrix elements $\overline{W}_{k\alpha}^{(0)}\left\{\left(\tilde{n}_j^{(0)}\left(t_i\right)\right)\right\}_{i+1}$, $\overline{W}_{\alpha k}^{(0)}\left\{\left(\tilde{n}_j^{(0)}\left(t_i\right)\right)\right\}_{i+1}$, $\overline{W}_{sm_j}^{(0)}\left\{\left(\tilde{n}_j^{(0)}\left(t_i\right)\right)\right\}_{i+1}$ and $\overline{W}_{m_j s}^{(0)}\left\{\left(\tilde{n}_j^{(0)}\left(t_i\right)\right)\right\}_{i+1}$. This leads with ongoing time to even improved "0-iteration," results as after one time step even the initial Boltzmann hypothesis (for the matrix elements) for the "0-iteration," is improved, in particular as usually the plasma parameters $n_e(t_i)$ and $T_e(t_i)$ are close to those of $n_e$ $(t_{i+1})$ and $T_e(t_{i+1})$.

Therefore, the present invention will allow further acceleration in transient cases. This will allow for the first time to perform even integrated simulations (hydro, radiation loss and spectral distributions) for very complex cases with spectroscopic precision that had been impossible up to now even for the largest computational resources.

The present invention of a new atomic physics splitting technique combined with a new split-iteration numerical procedure is for example devoted to the simulation of complex spectral distributions of matter with spectroscopic precision. The procedure enables numerical acceleration by many orders of magnitude and therefore allows to treat even the most complex atomic configurations like multiple excited states, hollow ions, ect. Applications are very wide concerning all type of radiation emission of atoms, ions, molecules, and dense matter with high spectroscopic precision for the spectral distribution and radiation losses.

The high convergence properties have been proven even for the 0-iteration thereby providing the possibility to accelerate additionally also numerical transient calculations. This will allow for the first time fully integrated simulations (coupling to hydro and atomic physics) while preserving spectroscopic precision.

A further outstanding property of the present invention is that at no step the exact solution according to the standard atomic physics theory is requested. Also the convergence is not obtained by refining the atomic system like in standard approaches but just by an additional iteration of the same split atomic system. Therefore even convergence studies do also not need the solution of the exact standard system. Thus is an enormous advantage when attacking problems where the exact atomic system cannot be established even not for a few benchmark simulations.

**System Implementations**

It is to be understood that embodiments of the present invention can be implemented in various forms of hardware, software, firmware, special purpose processes, or a combination thereof. In one embodiment, the present invention can be implemented in software as an application program tangible embodied on a computer readable program storage device. The application program can be uploaded to, and executed by, a machine comprising any suitable architecture.

More precisely, FIG. 5 is a block diagram of an exemplary computer system for efficiently performing a message passing algorithm. Referring now to FIG. 5, a computer system 51 for implementing the present invention can comprise, inter alia, a central processing unit (CPU) 52, a memory 53 and an input/output (I/O) interface 54. The computer system 51 is generally coupled through the I/O interface 54 to a display 55 and various input devices 56 such as a mouse and a keyboard. The support circuits can include circuits such as cache, power supplies, clock circuits, and a communication bus. The memory 53 can include random access memory (RAM), read only memory (ROM), disk drive, tape drive, etc., or a combinations thereof. The present invention can be implemented as a routine 57 that is stored in memory 53 and executed by the CPU 52 to process input data 58 (input data are the transition matrix elements $W_{xy}$ of the exact atomic system of eqs. 1.3-1.5 which are calculated beforehand, comprising, e.g., the calculations of cross sections and rate coefficients for a large interval of energies and temperatures). As such, the computer system 51 is a general purpose computer system that becomes a specific purpose computer system when executing the routine 57 of the present invention. The computer system 51 also includes an operating system and micro instruction code. The various processes and functions described herein can either be part of the micro instruction code or part of the application program (or combination thereof) which is executed via the operating system. In addition, various other peripheral devices can be connected to the computer platform such as an additional data storage device and a printing device.

It is to be further understood that, because some of the constituent system components and method steps depicted in the accompanying figures can be implemented in software, the actual connections between the process steps may differ depending upon the manner in which the present invention is programmed. Given the teachings of the present invention provided herein, one of ordinary skill in the related art will be able to contemplate these and similar implementations or configurations of the present invention.

The invention allows the reduction of the numerical burden by many orders of magnitudes to calculate non-equilibrium radiative properties with spectroscopic precision. The high convergence properties to the exact solution combined with the fact that the exact equations are never needed in any step of calculations will lead to an important impact in the simulations of the most complex properties of radiation and their technical implementation (computer code programming) of the method.

Numerous variations and modifications will become apparent to those skilled in the art once the above disclosure is fully appreciated. It is intended that the following claims be interpreted to embrace all such variations and modifications.

**Claims**

**1.** A method for efficiently determining a complex spectral high resolution distribution of a radiation source by determining populations of matter in atomic levels, the method comprising:

    using a computer to perform the following:

- considering an exact atomic system containing multiple excited levels "j" belonging to $M$ manifolds $\{\alpha\,(1)\}$ ... $\{\alpha\,(M)\}$, numerous ground and single excited levels "k", and corresponding transition matrix elements,
- performing a reduced system RS from the exact atomic system wherein all j- levels belonging to the manifolds $\{a\,(1)\}$ ... $\{\alpha\,(M)\}$ are lumped together to create one level for each manifold $\{\alpha\,(\beta)\}$, $\beta$=1 to M
- performing an Independent Recovery System of Equations IRSE containing j recovery systems computed from the exact atomic system, each recovery system comprising, instead of all j levels, a level j and a level $m_j$ obtained by lumping together all levels from the manifolds, except the j-level itself,
- calculating transition matrix elements $W_{\alpha jk}$ and $W_{k\alpha j}$ of RS to determine populations $n_k$ of all levels k,
- for each recovery system, calculating transition matrix elements $W_{mjk}$, $W_{kmj}$, $W_{mjj}$ and $W_{jmj}$ from $n_k$ to determine a non-statistical excited populations $n_j^{(\kappa)}$ of each level j, "$\kappa$" being an iteration index $\geq$0.

**2.** Method according to claim 1, wherein it further comprises iterative steps from index $\kappa$ =0..i..i$_{,max}$, "i$_{max}$" being the number of iterations of:

- inserting simultaneously into RS and IRSE the non- statistical excited populations $n_j^{(i-1)}$

- refining calculation of transition matrix elements $W_{\alpha jk}$ and $W_{k\alpha j}$ of RS from the non-statistical excited populations $n_j^{(i-1)}$,

- refining calculation of transition matrix elements $W_{mjk}$' $W_{kmj}$, $W_{mjj}$ and $W_{jmj}$ of IRSE from the non-statistical excited populations $n_j^{(i-1)}$.

**3.** Method according to claim 2, wherein after each iteration, the method further comprises determining if the non-statistical excited populations $n_j^{(i-1)}$ have converged.

**4.**

Method according to claim 3, wherein the method further comprises considering numerical values of $n_j^{(i-1)}$ to determine if the method has converged.

**5.** Method according to any of preceding claims, wherein during the calculation of transition matrix elements $W_{mjk}$, $W_{kmj}$, $W_{mjj}$ and $W_{jmj}$, the method further comprises determining weather the matrix elements and the level $m_j$ have converged.

**6.** Method according to any of preceding claims, wherein the iteration is accelerated by calculating refined populations $n_j^{(i)}$ not only from $n_j^{(i-1)}$ but from several preceding populations.

**7.** Method according to any preceding claims, wherein transition matrix elements $W_{jk}$ and $W_{kj}$ of RS are known from pure atomic physics calculations beforehand as no level lumping and populations are involved.

**8.** A non- transitory computer- readable storage medium encoded with computer- executable instructions which, when executed, perform a method for determining complex spatial distribution of a radiation source by determining populations of matter in atomic levels, the method comprising the steps of:

- considering an exact atomic system containing multiple excited levels "j" belonging to $M$ manifolds $\{\alpha\,(1)\}$ ... $\{\alpha\,(M)\}$, numerous ground and single excited levels "k", and corresponding transition matrix elements,
- performing a reduced system RS from the exact atomic system wherein all j- levels belonging to the manifolds $\{\alpha\,(1)\}$ ... $\{\alpha\,(M)\}$ are lumped together to create one level for each manifold $\{\alpha\,(\beta)\}$, $\beta$=1 to M
- performing an Independent Recovery System of Equations IRSE containing j recovery systems computed from the exact atomic system, each recovery system comprising, instead of all j levels, a level j and a level $m_j$ obtained by lumping together all levels from the manifolds, except the j-level itself,
- calculating transition matrix elements $W_{\alpha jk}$ and $W_{k\alpha j}$ of RS to determine populations $n_k$ of all levels k,
- for each recovery system, calculating transition matrix elements $W_{mjk}$, $W_{kmj}$, $W_{mjj}$ and $W_{jmj}$ from $n_k$ to determine a non-statistical excited populations $n_j^{(\kappa)}$ of each level j, "$\kappa$" being an iteration index $\geq$0.

FIGURE 1a

FIGURE 1b

FIGURE 2

Reduced system RS     Recovery system IRSE

FIGURE 3

Spectra for Titanium Z=22
Intensity [arb.units]

$T_e = 1$ keV
$He_\alpha$ $n_e = 10^{+23}$ cm$^{-3}$
Iteration = 0

Exact
Reduced

Y

0.261    0.2615    0.262    0.2625    0.263    0.2635
Wavelengths [nm]

FIGURE 4a

Spectra for Titanium Z=22
Intensity [arb.units]

$T_e = 1$ keV
$He_\alpha$ $n_e = 10^{+23}$ cm$^{-3}$
Iteration = 1

Exact
Reduced

Y

0.261    0.2615    0.262    0.2625    0.263    0.2635
Wavelengths [nm]

FIGURE 4b

51

52

CPU

55

DISPLAY

54

MEMORY

53

57

INPUT
DEVICE

56

DATA
INPUT

58

FIGURE 5

Europäisches
Patentamt

European
Patent Office

Office européen
des brevets

# EUROPEAN SEARCH REPORT

Application Number

EP 12 30 5392

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROSMEJ F. B.: "An alternative method to determine atomic radiative emission", EUROPHYSICS LETTERS: A LETTERS JOURNAL EXPLORING THE FRONTIERS OF PHYSICS, INSTITUTE OF PHYSICS PUBLISHING, BRISTOL, FR, vol. 76, no. 6, 1 December 2006 (2006-12-01), pages 1081-1087, XP020099068, ISSN: 0295-5075, DOI: 10.1209/EPL/I2006-10382-3 * the whole document * | 1-8 | INV. G06F19/00 |
| X | ROSMEJ F. B. ET AL: "Broadening and intensity properties of complex autoionizing states", JOURNAL OF QUANTITATIVE SPECTROSCOPY AND RADIATIVE TRANSFER, ELSEVIER SCIENCE, OXFORD, GB, vol. 99, no. 1-3, 1 May 2006 (2006-05-01), pages 548-559, XP028049574, ISSN: 0022-4073, DOI: 10.1016/J.JQSRT.2005.05.044 [retrieved on 2006-05-01] * the whole document * | 1-8 | |
| X | Rosmej F. B.: "Exotic States of High Density Matter Driven by Intense XUV/X-Ray Free Electron Lasers" In: "Free Electron Lasers", 14 March 2012 (2012-03-14), InTech, XP55036960, ISBN: 978-9-53-510279-3 pages 187-212, * the whole document * | 1-8 | TECHNICAL FIELDS SEARCHED (IPC)  G06F |

The present search report has been drawn up for all claims

| Place of search | Date of completion of the search | Examiner |
|---|---|---|
| The Hague | 28 September 2012 | Godzina, Przemyslaw |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **H.R. GRIEM.** Principles of Plasma Spectroscopy. Cambridge University Press, 1997
- **R.D. COWAN.** The Theory of Atomic Structure and Spectra. University of California Press, 1981
- **M.F. GU.** The flexible atomic code FAC. *Canadian Journal of Physics,* 2008, vol. 86 (5), 675
- **A. BURGESS.** *Astrophys. Journal,* 1964, vol. 139, 776
- **A. BAR-SHALOM ; J. OREG ; W.H. GOLDSTEIN ; D. SHVARTS ; A. ZIGLER.** *Phys. Rev.,* 1989, vol. A 40, 3183